# EUROPÄISCHE PATENTSCHRIFT

(11) **EP 0 770 116 B1**
(45) Veröffentlichungstag und Bekanntmachung des Hinweises auf die Patenterteilung: **22.09.1999**
(21) Anmeldenummer: 95924847.7
(22) Anmeldetag: 11.07.1995
(51) Int. Cl.: C09K 9/02, C07D 311/92, G03C 1/685

(54) **DIARYL-2H-NAPHTHOPYRANE**
DIARYL-2H-NAPHTHOPYRANES
DIARYLE-2H-NAPHTOPYRANNES

(30) Priorität: 11.07.1994 DE 4424351
(43) Veröffentlichungstag der Anmeldung: 02.05.1997
(73) Patentinhaber: Optische Werke G. Rodenstock, 80469 München (DE)
(72) Erfinder: MELZIG, Manfred, D-82234 We ling (DE); ZINNER, Herbert, D-82024 Taufkirchen (DE)
(74) Vertreter: Münich, Wilhelm, Dr.
(86) Internationale Anmeldenummer: DE9500897
(87) Internationale Veröffentlichungsnummer: WO9601884

(56) Entgegenhaltungen:
- WO-A-95/16215
- US-A- 5 238 981

## Beschreibung

### Technisches Gebiet

Die Erfindung bezieht sich auf photochrome Diaryl-2H-naphthopyrane.

### Stand der Technik

Pyrane sind eine seit über 25 Jahren bekannte Klasse photochromer Verbindungen. Eine der ersten Beschreibungen von Pyranen ist in der US-PS 3 567 605 zu finden, in der verschiedene Benzo- und Naphthopyrane, darunter auch Diphenyl-naphthopyrane beschrieben sind. Diese Verbindungen blieben jedoch über Jahrzehnte uninteressant, da sie nur bei sehr tiefen Temperaturen (-40°C) einfärben.

Erst seit kurzer Zeit sind Pyrane bekannt, die auch bei Normaltemperaturen photochrom einfärben und damit für eine Verwendung in Brillengläsern geeignet erscheinen. Hierzu wird exemplarisch auf die US-PS 5 066 818, die WO 93/10112 oder die WO 92/09593 verwiesen.

Diese bekannten Pyrane weisen jedoch noch Unzulänglichkeiten hinsichtlich der Lebensdauer und/oder der Migrationseigenschaften, sowie ihrer photochromen Eigenschaften auf.

Insbesondere die stoffspezifisch unterschiedliche Migration der einzelnen Pyran-Verbindungen führt zu unzureichenden Färbe-Ergebnissen bei der Oberflächenfärbung, wenn diese Pyran-Verbindungen in Mischungen, die zur Erzielung eines gewünschten Mischfarbtons nötig sind, eingesetzt werden, da jede einzelne Verbindung unterschiedlich tief, in Abhängigkeit von der spezifischen Migrationsgeschwindigkeit, in das einzufärbende Material eindringt.

Dadurch ergeben sich, vergleichbar mit den Vorgängen bei der Chromatographie, Schichten, in denen die unterschiedlichen Pyran-Verbindungen in überdurchschnittlichen Konzentrationen aufzufinden sind.

Außerdem resultiert aus der relativ großen Migrationsgeschwindigkeit der aus dem Stand der Technik bekannten Verbindungen eine kurze Haltbarkeit der eingefärbten Kunststoffgegenstände. Die bekannten Farbstoffe migrieren vergleichsweise schnell aus dem eingefärbten Gegenstand an dessen Oberläche und führen dort zu Ablöseerscheinungen.

### Darstellung der Erfindung

Der Erfindung liegt die Aufgabe zugrunde, Substanzen anzugeben und deren Synthese zu beschreiben, die bei Raumtemperatur gut photochrom einfärben, für ein Brillenglas ausreichend schnell aufhellen, eine brauchbare Lebensdauer und aufgrund ihrer räumlichen Struktur eine geringere Migrationsneigung als die aus dem Stand der Technik bekannten Verbindungen aufweisen.

Eine erfindungsgemäße Lösung dieser Aufgabe ist in den Patentansprüchen 1 und 2 angegeben. Weiterbildungen der Erfindung sind Gegenstand der abhängigen Ansprüche.

Gegenstand der Erfindung sind damit photochrome Verbindungen mit Diaryl-2H-naphthopyran-Struktur, die an ihren Arylsubstituenten eine vorteilhafte Substitution aufweisen.

Diese Substitution der Arylsubstituenten besteht einerseits aus der Verknüpfung des Naphthopyran-Gerüstes mit der im Patentanspruch 2 unter a) angegebenen ersten Ausgangsverbindung durch das erfindungsgemäße Herstellverfahren, wobei 'monomere' Diaryl-2H-naphthopyrane erhalten werden, die ein photochrom reagierendes Zentrum aufweisen.

Andererseits weist die durch das beschriebene Verfahren erhaltene photochrome Substanz eine besonders vorteilhafte Substitution der Arylsubstituenten auf, wenn der Arylsubstituent der Diaryl-2H-naphthopyran-Verbindung über die im Patentanspruch 2 unter a) angegebene Ausgangsverbindung mit wenigstens einer weiteren Diaryl-2H-naphthopyran-Verbindung verknüpft ist.

Abhängig von der Wahl der Ausgangsverbindung 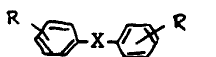 weist die photochrome Substanz eine Verknüpfung von wenigstens zwei photochromen Teilsystemen auf, die entweder durch eine elektronenleitende, oder durch eine die Elektronendelokalisation unterbrechende Brücke gekennzeichnet ist.

Erfindungsgemäß ist nämlich erkannt worden, daß eine Lösung der gestellten Aufgabe in sehr einfacher Weise dadurch gelingt, daß die Arylsubstituenten der aus dem Stand der Technik bekannten Diaryl-2H-naphthopyran-Verbindungen in geeigneter Weise substituiert werden.

Dabei bleiben alle photochromen Eigenschaften, wie Farbe, Eindunkelungs- und Aufhellgeschwindigkeit, Temperaturabhängigkeit und Lebensdauer der photochromen Reaktion erhalten; gleichzeitig zeigen die erfindungsgemäßen Verbindungen eine deutlich geringere Migrationsneigung als die aus dem Stand der Technik bekannten Verbindungen.

Darüber hinaus lassen sich auf diesem Weg verschiedene Diaryl-2H-Naphthopyrane miteinander verknüpfen. Dies hat den Vorteil, daß mehrere Farbstoffe unterschiedlicher Absorption im unangeregten wie im angeregten Zustand identische Migrationsfähigkeit aufweisen, da sie sich in einem Gesamtmolekül befinden. Dadurch können verschiedene Probleme, die mit unterschiedlichen Farbstoffen gerade bei der Oberflächenfärbung auftreten, elegant vermieden werden.

### Beschreibung von Ausführungsbeispielen

Die Erfindung wird nachstehend anhand von Ausführungsbeispielen näher beschrieben.

Zunächst soll die Synthese der photochromen Substanzen beschrieben werden:
1. Herstellung der Ausgangsprodukte zur Synthese eines Ketons gemäß Anspruch 2 a):
   Zur Synthese der erfindungsgemäßen Substanzen wird von einer Diarylverbindung ausgegangen. Diese kann ein Diarylalkan und/oder ein Diarylether aus den im Anspruch 2 a) angegebenen homologen Reihen sein.
   a) Synthese der Diarylalkane:
      Die kurzkettigen Homologen dieser Ausgangsverbindung, Biphenyl, Diphenylmethan und Dibenzyl (n = 0, 1, 2), sind über den Chemikalienhandel, beispielsweise über Aldrich (Nr. 14410, 43100) zu erwerben.
      Die Ausgangsverbindung 1,3-Diphenylpropan (n = 3) ist über die Clemmsen-Reduktion des käuflichen Dibenzylketons am einfachsten zu herzustellen.
      Hierzu werden 30 g Zinkstaub, 2,5 g HgCl₂, 1,5 ml konz. HCl und 38 ml Wasser fünf Minuten geschüttelt. Die überstehende Flüssigkeit wird abdekantiert, dann werden dem gebildeten Amalgam 20 ml Wasser, 45 ml konz. HCl, 25 ml Toluol und 14,7 g Dibenzylketon zugegeben. Diese Mischung wird 24 Stunden unter Rückfluß erhitzt, wobei in Abständen von 5 Stunden je 12,5 ml konzentrierte HCl zugegeben werden. Nach dem Abkühlen wird die organische Phase abgetrennt, die wässrige Phase wird mit 50 ml Wasser verdünnt und 3 mal mit je 50 ml Ether extrahiert. Die Etherphasen werden vereinigt und mit 50 ml Wasser ausgeschüttelt, anschließend über Na₂SO₄ getrocknet. Nach dem Abziehen des Ethers verbleibt die Ausgangsverbindung 1,3-Diphenylpropan als farbloses Öl.
      Zur Herstellung des nächsthöheren Homologen mit n = 4, dem 1,4-Diphenylbutan ist die Hydrierung von über den Handel erhältlichem 1,4-Diphenyl-1,3-Butadien geeignet. Dazu werden 21,5 g 1,4-Diphenyl-1,3-Butadien in 400 ml Eisessig suspendiert und unter Einleitung von H₂-Gas mit 8 g Paladiumkohle, entsprechend einer Menge von etwa 0,5 g Paladium, versetzt. Die Hydrierung ist abgeschlossen, wenn sich das Butadien vollständig aufgelöst hat und die schwach violette Fluoreszenz erlischt. Nach der Filtration wird das Filtrat bis zur Trübung mit Wasser versetzt und 10 Minuten auf 85° C erhitzt. Anschließend wird abgekühlt und mit Toluol ausgeschüttelt. Die Toluolphase wird über Na₂SO₄ getrocknet, das Lösemittel abgezogen und der Rückstand destilliert. Die gewünschte Ausgangsverbindung, 1,4-Diphenylbutan, geht bei der Destillation als farbloses Öl über, das noch in der Vorlage zu einer weißen Masse erstarrt. Der Schmelzpunkt liegt bei 52°C.
      Zur Herstellung des 1,5-Diphenylpentans (n = 5) wird die Verbindung 1,5-Diphenyl-1,4-pentadien-3-on, die über Aldrich unter der Nummer 43143 zu beziehen ist, hydriert. Dazu werden 50 g des Ketons in 500 ml Aceton bei einer Reaktionstemperatur von 15° C mit 16 g Paladiumkohle versetzt. Während der unter Einleitung von H₂-Gas von selbst ablaufenden Hydrierung färbt sich die überstehende Flüssigkeit hellgrün. Nach abgeschlossener Hydrierung wird filtriert. Dem Filtrat wird das Lösemittel abgezogen und der Rückstand anschließend destilliert. Bei 185°C und einem Druck von 2666 Pa geht die gewünschte Verbindung, 1,5-Diphenylpentan als farbloses öl über. Die Ausbeute ist praktisch quantitativ.
      Zur Herstellung der Ausgangsverbindung mit n = 6, dem 1,6-Diphenylhexan ist beispielsweise die Hydrierung von 1,6-Diphenylhexatrien geeignet. Dazu werden 24 g Diphenylhexatrien, das über Aldrich unter der Nummer 43050 zu beziehen ist, in 400 ml Eisessig suspendiert und mit 8 g paladiumkohle, die in etwa einer Menge von 0,5 g Paladium entsprechen, versetzt. Die H₂-Einleitung wird bis zur vollständigen Auflösung des Triens und dem Erlöschen der blauen Fluoreszenz fortgesetzt. Nach anschließender Filtration wird das Filtrat bis zur Trübung mit Wasser versetzt und 10 Minuten auf 85° C erhitzt. Dabei scheidet sich an der Oberfläche ein farbloses Öl ab, das nach Abtrennung destilliert wird. Die zur Synthese der erfindungsgemäßen Substanzen gewünschte Ausgangsverbindung, 1,6-Diphenylhexan, geht bei der Destillation bei 208° C und einem Druck von 2666 Pa über. Das farblose Öl erstarrt nur sehr schwierig. Die aus Ethanol erhältlichen weißen Kristalle weisen einen Schmelzpunkt von 136 bis 137° C auf.
   b) Synthese der Diarylether:
      Die zur Synthese der erfindungsgemäßen photochromen Substanz mit Diaryl-2H-Naphthopyranstruktur eingesetzten symmetrischen Diarylether, wie Diphenylether (m=n=0) und Dibenzyl-ether (m=n=1) sind über Aldrich unter den Nummern 42730 und 33630 zu beziehen. Die Darstellung des Diphenethylethers (m=n=2) erfolgt nach den Angaben bei Jain et al., J.Ind. Chem. Soc. 28 (1951), 49; der Bis-(3-phenylpropyl)-ether (m=n=3) kann nach einer Vorschrift in A. Morton et al., J.A.C.S. 63 (1941) 326 hergestellt werden.
      Die ebenso als Ausgangsverbindung geeigneten unsymmetrischen Ether sind durch nachfolgend beschriebene Synthesen leicht darzustellen.
      Zur Herstellung des Benzylphenethylethers (m=1, n=2) werden 23 g feinster Na-Draht in 350 ml Toluol suspendiert. Anschließend wird unter gutem Rühren tropfenweise 122 g Phenethylalkohol zugegeben. Während dieser Zugabe wird die Mischung zum Sieden erhitzt, wobei nach etwa 1 Stunde die Gasentwicklung aufhört. Nach anschließender Zugabe von 126 g Benzylchlorid wird noch weitere 90 Minuten unter Rückfluß gekocht. Danach wird abgekühlt und mit Wasser zersetzt. Von den entstehenden zwei Phasen wird die organische Phase abgetrennt, mit Wasser gewaschen, getrocknet und fraktioniert destilliert. Bei der Destillation geht der als Ausgangsverbindung für die Synthese der photochromen Substanz zu verwendende Benzylphenethylether bei etwa 180° C und 1733 Pa als farbloses Öl über.
   c) Synthese von am Arylrest substituierten Diarylverbindungen:
      Zur Herstellung von 1-Phenyl-3-(3,4,5-trimethoxyphenyl-) propan als Ausgangsprodukt für die Synthese der photochromen Substanz mit Diaryl-2H-Naphthopyranstruktur, werden 81 g 3,4,5-Trimethoxybenzaldehyd, 49 g Acetophenon, 150 ml Ethanol und 150 ml 10%-ige NaOH 4 Stunden bei Raumtemperatur gerührt. Der Aldehyd und das Phenon sind über Aldrich unter den Nummern 92140 und 00790 erhältlich. Nach dem Abkühlen auf unter 5°C werden die ausgefallenen gelben Kristalle (Smp. 131°C) aus Ethanol umkristallisiert. 60 g dieses Produkts werden in 400 ml Ethylacetat suspendiert, mit 30 g Paladiumkohle als Katalysator und 4 ml 70%-ige Perchlorsäure versetzt und im Autoklaven unter Rühren 30 Std. mit 2,5 bar H2 hydriert. Das Gemisch wird filtiert und das Filtrat anschließend destilliert. Bei einem Druck von 66,6 Pa und 180 bis 196°C geht das Syntheseprodukt 1-Phenyl-3-(3,4,5-trimethoxyphenyl-)propan als farbloses Öl über.
      Aufgrund der Vielzahl der im Handel erhältlichen substituierten Benzaldehyde und Acetophenone, sowie der leichten Durchführbarkeit und hohen Ausbeute der beschriebenen Reaktion (meist 70%) ist dieser Weg auch für alle anderen hier nicht explizit beschriebenen Ausgangsverbindungen zur Synthese der erfindungsgemäßen Substanz denkbar.
      Zur Synthese der im Arylteil substituierten Diarylether werden equimolare Mengen Benzylchlorid oder -bromid, die in geeigneter Weise substituiert sein können, und Phenol mit einer equimolaren Menge Natriumethylat in 5%-igem Ethanol so lange auf dem Wasserbad gekocht, bis die Reaktionslösung neutral geworden ist. Hierbei kann das Produkt von selbst ausfallen; ist dies nicht der Fall, muß Alkohol abgezogen werden, um das Ausfallen zu erzwingen. Anschließend wird abfiltriert, mit Wasser nachgewaschen und umkristallisiert. Erhält man, bedingt durch die Substitution des Benzylchlorids, -bromids oder des Phenols ein öliges Produkt, so wird die neutrale Reaktionslösung stark mit Wasser verdünnt und ausgeethert. Der erhaltene Etherauszug wird mit verdünnter 2%-iger NaOH gewaschen, über Na₂SO₄ getrocknet und das Lösungsmittel abgezogen. Für den Fall, daß das Produkt nach dieser Behandlung immer noch ölige Konsistenz aufweist, wird es im Hochvakuum einer fraktionierten Destillation unterworfen (133 bis 67 Pa). Wird beispielsweise 2,4-Dibromphenol, das über Aldrich, Nr. 34270 erhältlich ist, mit Benzylbromid und einer equimolaren Menge Natriumethylat in 5%-igem Ethanol zur Reaktion gebracht, so erhält man 2,4-Dibromphenyl-benzylether. Nach der Umkristallisation aus Ethanol/Wasser werden farblose glänzende Nadeln mit einem Schmelzpunkt von 68°C erhalten.
      Der substituierte Diarylether, 2,3,5-Trimethylphenyl-benzylether, kann hergestellt werden, indem 2,3,5-Trimethylphenol mit Benzylbromid unter obengenannten Bedingungen zur Reaktion gebracht wird. In diesem Fall werden nach der Umkristallisation flache farblose Prismen mit einem Schmelzpunkt von 45°C erhalten.
2. Synthese der Ketone gemäß Anspruch 2 a):
   a) Synthese von 1,2-Di(4-benzophenonyl-)ethan und 4-Phenethylbenzophenon:
      Zu 181,4 g (1,36 mol) wasserfreiem AlCl₃ in 400 ml 1,2-Dichlorethan gibt man unter Rühren bei Raumtemperatur 168,8 g (1,20 mol) Benzoychlorid. Die Suspension färbt sich unter Auflösung des Aluminiumsalzes dunkler. Unter schwacher Kühlung gibt man bei < 20°C 100 g (0,56 mol) Dibenzyl zu. Die dunkelbraune Lösung wird noch 1 h gerührt und über Nacht stehen gelassen. Man gießt die Reaktionsmasse vorsichtig auf 400 g Eis und säuert mit HCl an. Die organische Phase wird abgetrennt, mit 2%iger NaOH ausgeschüttelt und mit Natriumsulfat getrocknet. Nach dem Filtrieren wird das Dichlorethan abgezogen. Man erhält 160,1 g eines orangen Öls, das neben etwas Ausgangsprodukt zu 82 % aus 1,2-Di(4-benzophenon)-ethan und 14 % aus 4-Phenethyl-benzophenon besteht.
   b) Synthese eines im Arylteil substituierten Ketons:
      Zu 66 g wasserfreiem Alcl ₃ in 100 ml Schwefelkohlenstoff gibt man unter absolutem Feuchtigkeitsausschluß unter gutem Rühren 62 g Benzoylchlorid. Anschließend werden 30 g Dimethylaminobiphenyl in 300 ml Schwefelkohlenstoff zugefügt und das Reaktionsgemisch 15 Std. unter Rückfluß gekocht. Die Reaktionsmasse wird anschließend vorsichtig auf Eis gegossen und mit HCl angesäuert. Die organische Phase wird abgetrennt und mit NaOH versetzt. Das sich daraufhin abscheidende Produkt wird abfiltiert und aus Ethylmetylketon umkristallisiert. Nach dem Umkristallisieren werden goldgelbe Plättchen mit einem Smp. von 180° C erhalten.
   c) Synthese eines Ketons aus einem Diarylether:
      Ein unter 1. b) erhaltener Diarylether wird bei etwa 10 bis 15° C in 1,2-Dichlorethan 110 g vorgelegt.
      Anschließend werden 180 g wasserfreies AlCl₃ und 170 g Benzoylchlorid in 400 ml 1,2-Dichlorethan tropfenweise hinzugefügt.
      Die für Friedel-Crafts-Acylierungen ungewöhnlich geringen Überschüsse an AlCl₃ sind im Falle der oben beschriebenen Umsetzungungen der Diarylether mit den Arylcarbonsäure-halogeniden strikt einzuhalten, da sonst leicht Etherspaltung eintritt.
      Bei der Einführung von Methoxy- oder Ethoxygruppen über den Arylrest des Säurehalogenids findet die Etherspaltung in viel geringerem Maße statt, so daß diese Ketone problemlos hergestellt werden können.
3. Synthese des tert. Alkohols bzw. des Carbinols:
   160 g der Mischung aus 2. a) werden in 350 ml getrocknetem Dimethylsulfoxid unter Rühren gelöst und 78 g Lithium-acetylid-ethylendiaminkomplex langsam zugegeben. Die dunkelbraune Lösung wird 62 h bei Raumtemperatur gerührt, wobei sie sich nach tiefrot verfärbt. Sie wird vorsichtig auf 800 g Eis gegeben und sofort mit 2n HCl angesäuert. Die Reaktionsmasse wird im Flüssig-flüssig-extraktor mit Ether extrahiert bis die überstehende Etherphase völlig farblos ist. Nach dem Trocknen des Ethers mit Natriumsulfat wird filtriert, dem Filtrat der Ether entzogen. Zurück bleiben 171,4 g eines orangen Öls, das sich nach längerem Stehenlassen zu einer gelben Kristallmasse verfestigt. Diese besteht zu 74 % aus 4,4'-Di(1-Hydroxy-1-phenyl-prop-3-in-1-yl)dibenzyl und zu 13 % aus 3-Phenyl-3-(4-Phenethyl)phenyl-prop-1-in-3-ol.
4. Synthese der photochromen Verbindungen:
   109 g der Mischung aus 3. werden in 500 ml Toluol gelöst und 73,2g 6-Methoxy-2-naphthol zugegeben. Die dunkelgelbe Lösung wird unter Rühren auf 60°C erhitzt, nach Zugabe von einer Spatelspitze 4-Toluolsulfonsäure wird sie schnell dunkler. Die Papierprobe unter UV-Licht ergibt sofort orange Photochromie. Die Lösung wird noch 1 h bei 60°C gerührt. Nach dem Abziehen des Toluols wird das zurückbleibende grüne Öl in Ethanol aufgenommen, kurz aufgekocht und über Nacht stehen gelassen. Nach dem Abtrennen von Unlöslichem wird das Ethanol abgezogen und der Rückstand über Aluminiumoxid mit Methylenchlorid chromatographiert. Von den 4 entstandenen orangen photochromen Verbindungen ( DC-Kontrolle ) werden nur die 2 in der ersten Fraktion befindlichen aufgefangen. Nach dem Abziehen des Lösemittels verbleiben 66 g eines orangen Öls. Dieses kristallisiert nach einigen Tagen zu einer gelben kristallinen Masse aus. Diese kann nach dem Waschen mit Methanol zur Einfärbung von Kunststoffgläsern verwendet werden. Durch fraktionierte Kristallisation ( Lösen der Kristalle in Diethylether und teilweise Ausfällung mit Ethanol; Vorgang mehrfach wiederholen ) lassen sich die beiden photochromen Verbindungen getrennt gewinnen.

Die Hauptmenge besteht nach NMR-Analyse aus: 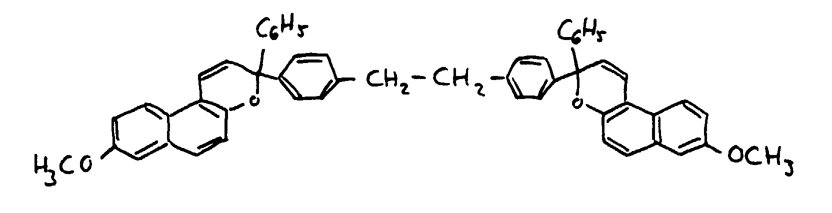 Die zweite Verbindung wird als 'monomere' photochrome Substanz mit der folgenden Struktur: 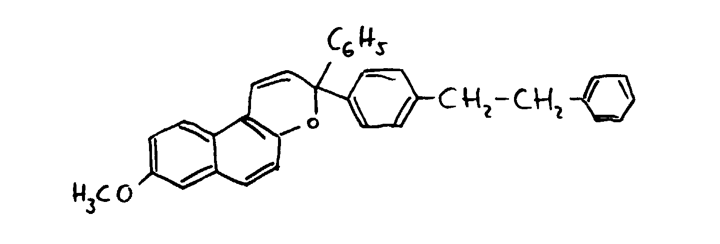 identifiziert.

Vergleich der erfindungsgemäßen photochromen Substanzen mit dem Substanzen nach dem Stand der Technik:
Nach den unter 1. bis 4. angegebenen Synthesevorschriften werden erfindungsgemäße Substanzen entsprechend folgender Strukturformel mit den bezeichneten Resten hergestellt: 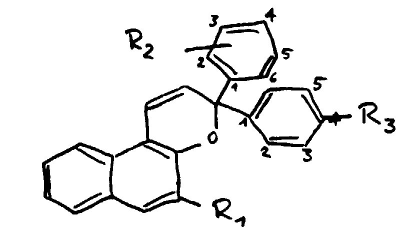 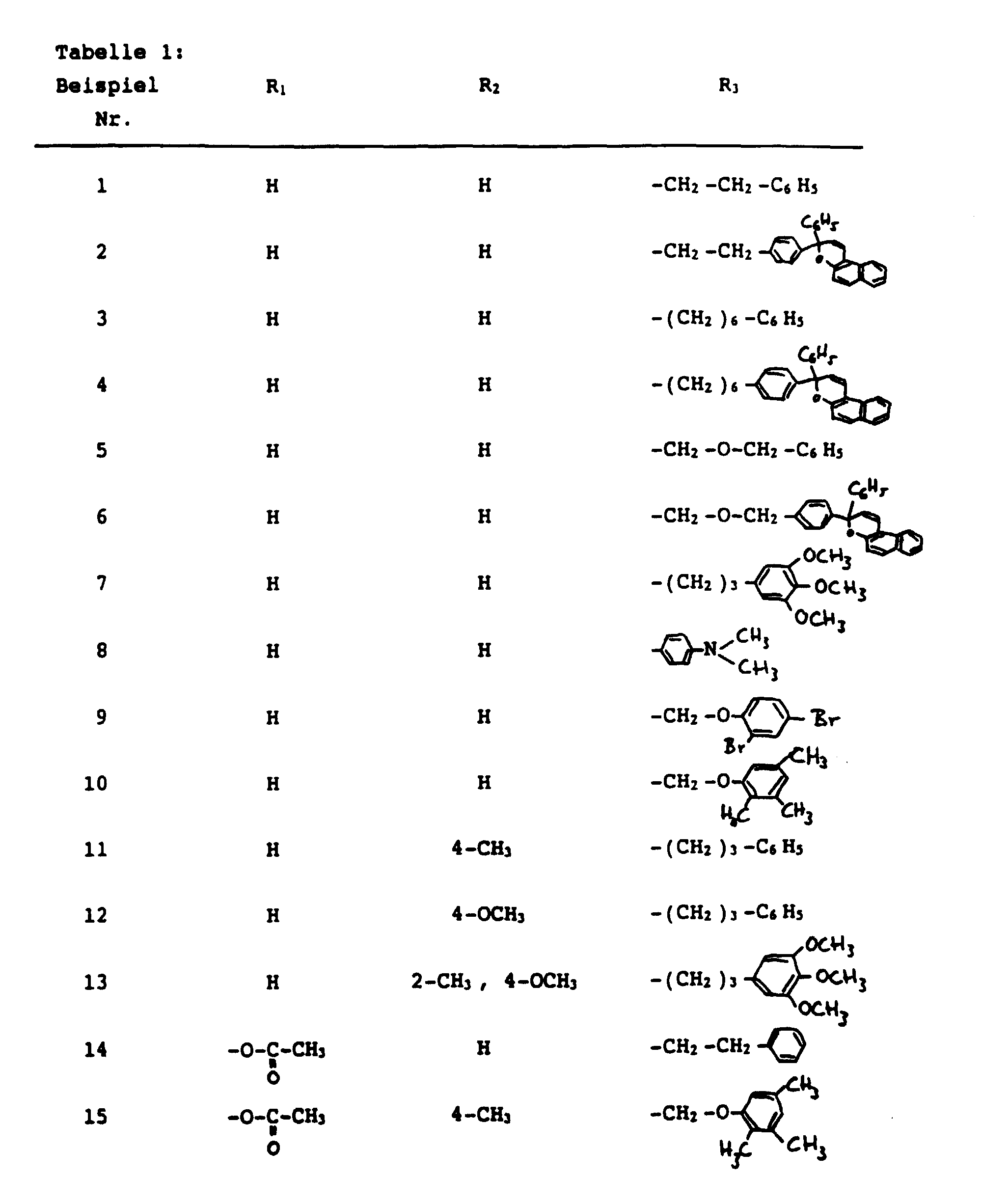 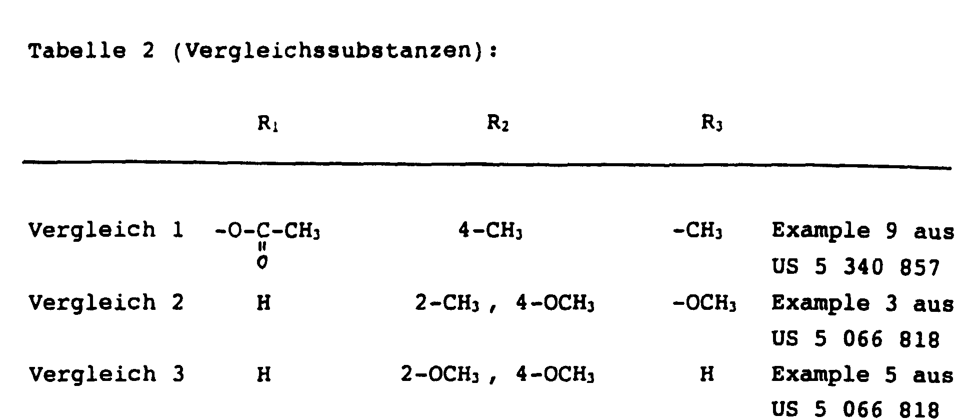 Mit den erhaltenen Substanzen und mit den drei Vergleichssubstanzen aus dem Stand der Technik werden je zehn oberflächengefärbte und superentspiegelte Gläser aus CR 307 (eingetragenen Warenzeichen von PPG Industries) hergestellt.

Diese werden, um das Migrationsverhalten der photochromen Substanzen und der Vergleichssubstanzen zu beurteilen, nach einer bestimmten Anzahl von Kochzyklen von jeweils 2 min in 4 % iger NaCl-Lösung untersucht. Dabei wird festgestellt, bei welcher Anzahl der ursprünglichen zehn Gläser nach dem bezeichneten Kochzyklus Ablöseerscheinungen auftreten.

Diese Ablöseerscheinungen, die durch das Migrieren der mittels Oberflächenfärbung eingebrachten photochromen Substanzen an die Oberfläche des eingefärbten Gegenstandes und deren nachfolgendes Abblättern gekennzeichnet sind, treten bei photochromen Substanzen mit stärkeren Migrationseigenschaften schneller auf, da die Migrationsgeschwindigkeit größer ist.

**Tabelle 3:**

| | Anzahl der Kochzyklen | | | | | | | | | | |
|---|---|---|---|---|---|---|---|---|---|---|---|
| | 1 | 2 | 3 | 4 | 5 | 6 | 7 | 8 | 9 | 10 | >10 |
| Beispiel Nr. | | | | | | | | | | | |
| 1 | - | - | 2 | 3 | 3 | 2 | | | | | |
| 2 | - | - | - | - | 1 | 3 | 2 | 2 | 1 | - | 1 |
| 3 | - | - | - | 3 | 3 | 2 | 2 | | | | |
| 4 | - | - | - | - | 1 | 2 | 1 | 3 | - | 1 | 2 |
| 5 | - | - | 2 | 2 | 4 | 1 | 1 | | | | |
| 6 | - | - | - | - | 2 | 2 | 1 | 2 | 1 | - | 2 |
| 7 | - | - | - | 1 | 3 | 3 | 1 | 1 | 1 | | |
| 8 | - | - | 2 | 4 | 2 | 2 | | | | | |
| 9 | - | - | 1 | 2 | 3 | 3 | 1 | | | | |
| 10 | - | - | 1 | 3 | 4 | 2 | | | | | |
| 11 | - | - | - | - | 1 | 1 | 2 | 2 | 2 | - | 2 |
| 12 | - | - | - | - | 1 | - | 2 | 3 | 3 | - | 1 |
| 13 | - | - | - | - | 2 | 3 | 3 | - | 1 | 1 | |
| 14 | - | - | 2 | 4 | 2 | 1 | 1 | | | | |
| 15 | - | - | - | 2 | 3 | 3 | 1 | 1 | | | |

| Vergleich Nr. | | | | | | | | | | | |
|---|---|---|---|---|---|---|---|---|---|---|---|
| 1 | - | 3 | 4 | 2 | 1 | | | | | | |
| 2 | - | 1 | 3 | 3 | 2 | 1 | | | | | |
| 3 | 1 | - | 4 | 2 | 3 | | | | | | |

Aus diesen Beispielen ist zu erkennen, daß die mit den erfindungsgemäßen Substanzen eingefärbten Gegenstände erst nach einer größeren Anzahl Kochzyklen Ablöseerscheinungen aufweisen. Keines der mit den Beispielsubstanzen 1 bis 15 eingefärbten Gläser zeigt nach den ersten beiden zweiminütigen Kochzyklen in 4 % iger NaCl-Lösung diese durch größere Migrationsgeschwindigkeit verursachten Ablösungen. Von den mit den Vergleichssubstanzen 1 bis 3 eingefärbten Gläsern zeigt eines schon nach dem ersten Kochzyklus Ablösung, vier der Gläser (drei mit Vergleich Nr. 1 und eines mit Vergleich Nr. 2) bereits nach dem zweiten.

## Patentansprüche

1. Photochrome Substanz mit Diaryl-2H-naphthopyran-struktur, die in der Form Y-Ar-Y, Y-Ar-Y', Y-Ar-Z, Z-Ar-Z oder Z-Ar-Z' miteinander verknüpft sind, wobei Y, Y', Z und Z' Pyrane sind.

2. Photochrome Substanz mit Diaryl-2H-naphthopyran-struktur,
dadurch **gekennzeichnet**, daß sie einen Arylsubstituenten aufweist, der die Migration der Moleküle in
Kunststoffen verringert, erhalten durch:
a) Synthese eines Ketons aus einem Diarylalkan und/oder einem Diarylether und einem Arylcarbonsäurehalogenid durch Friedel-Crafts-Acylierung nach folgendem Reaktionsschema: 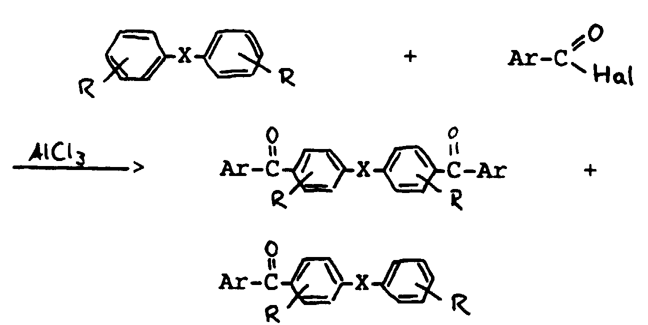 mit:
X = -(CH₂)ₙ- n = 0,1,2,..,6
-(CH₂ )ₙ-O-(CH₂)ₘ- n = 0,1,2,3
m = 0,1,2,3
R = H, F, Cl, Br, Phenyl, Benzyl, OR', R',NR'₂
R'= Alkyl mit bis zu 6 C-Atomen oder Cycloalkyl mit 5 oder 6 C-Atomen
Ar = 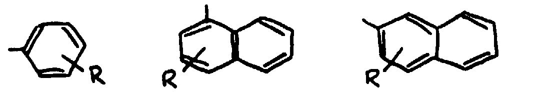
b) Synthese eines tert. Alkohols mit Kohlenstoff-Kohlenstoff-Dreifachbindung aus durch a) erhaltenem Keton und Metallacetylid durch nukleophile Addition.
c) Synthese der photochromen Substanz durch Kondensation des tert. Alkohols mit einem substituierten Diarylalkohol nach folgendem Reaktionsschema: 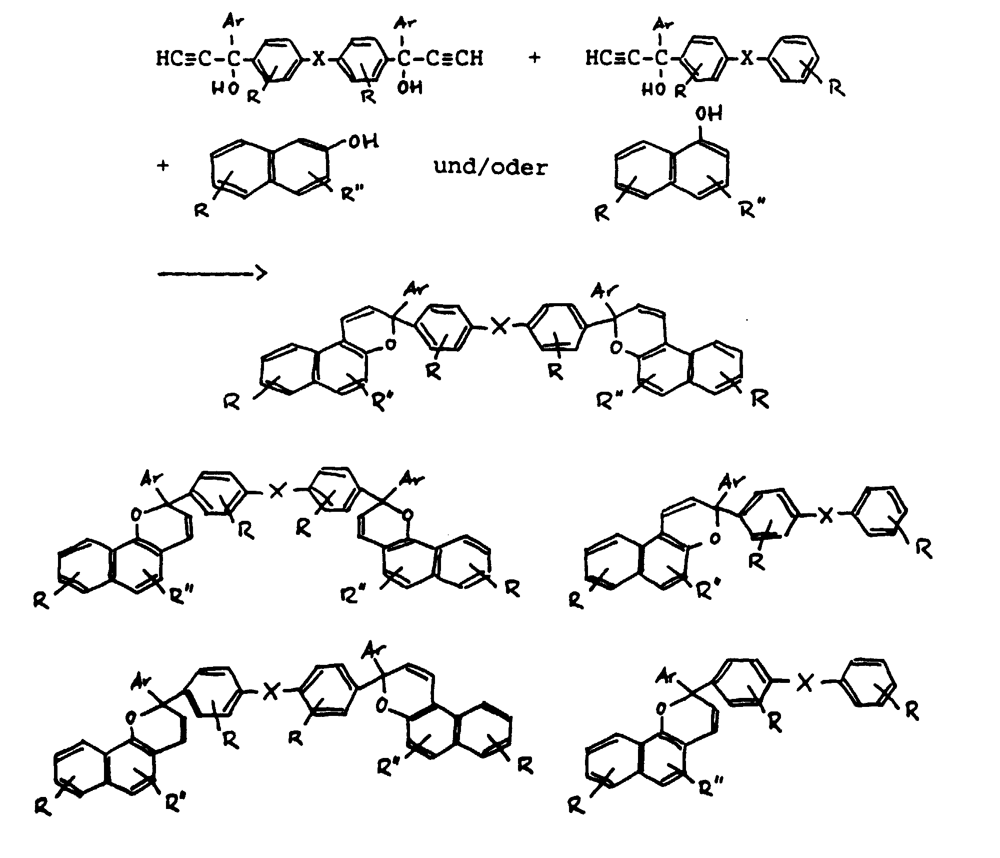 mit:
X, Ar, R, R'wie unter a)
R'' = H, F, Cl, Br, R', OR',

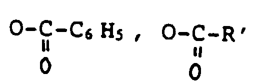

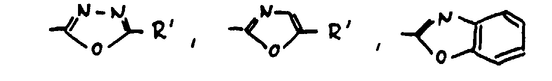

3. Photochrome Substanz nach Anspruch 2 oder 3, dadurch **gekennzeichnet**, daß sie zur Einfärbung von Brillengläsern aus einem Kunststoffmaterial geeignet ist.

## Claims

1. Photochromic substance with a diaryl-2H-naphthopyrane structure, which are linked to one another in the form Y-Ar-Y, Y-Ar-Y', Y-Ar-Z, Z-Ar-Z or Z-Ar-Z', wherein Y, Y', Z and Z' are pyrans.

2. Photochromic substance with a diaryl-2H-naphthopyrane structure,
**characterised in that** it has an aryl substituent which lessens the migration of the molecules in plastics, obtained by:
a) Synthesis of a ketone from a diarylalkane and/or a diarylether and an aryl carboxylic acid halide by Friedel-Crafts acylation according to the following reaction scheme: 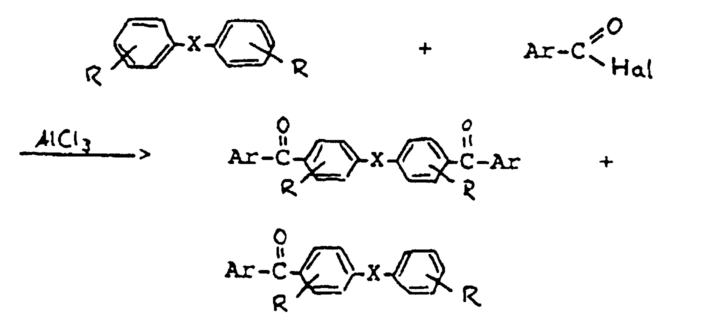 with
X = -(CH₂)ₙ- n = 0,1,2,..,6
-(CH₂)ₙ-O-(CH₂)ₘ- n = 0,1,2,3
m = 0,1,2,3
R = H, F, Cl, Br, Phenyl, Benzyl, OR', R',NR'₂
R'= alkyl with up to 6 C-atoms or cycloalkyl with 5 or 6 C-atoms
Ar = 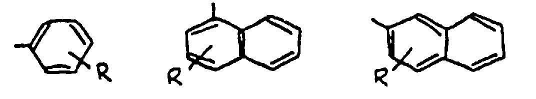
b) Synthesis of a tert. alcohol with a carbon-carbon triple link from ketone and metal acetylide obtained through a) by nucleophilic addition.
c) Synthesis of the photochromic substance by condensation of the tert. alcohol with a substituted diarylalcohol according to the following reaction scheme: 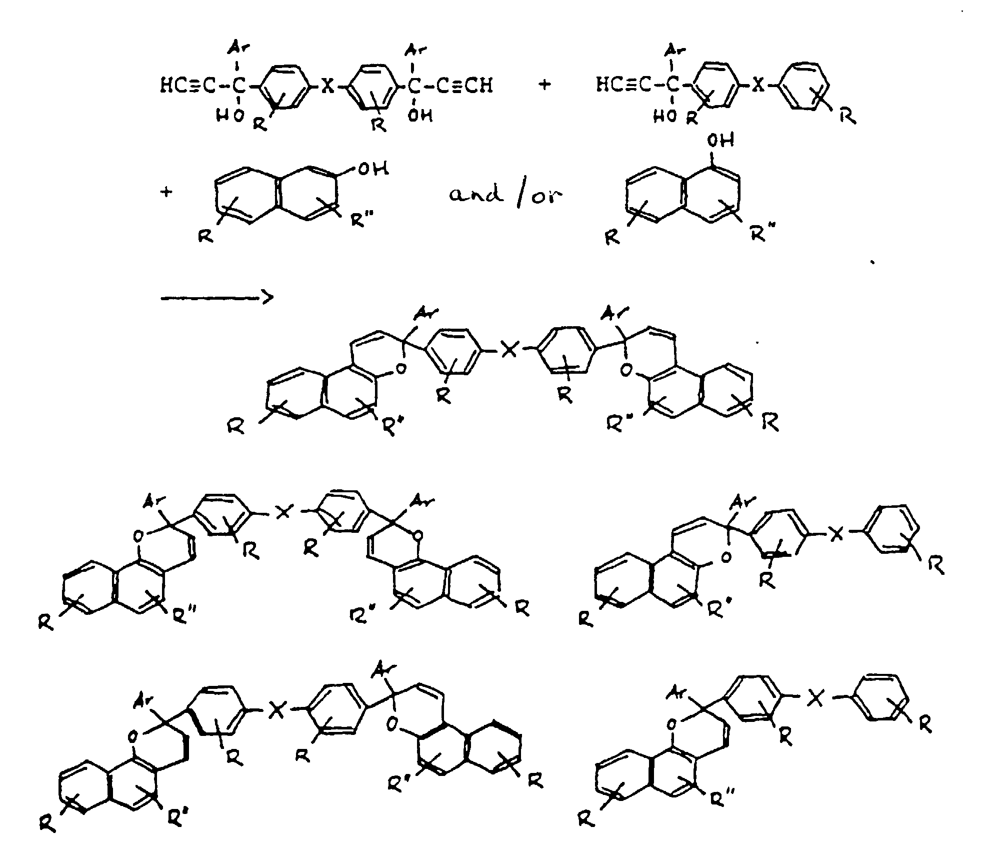 with:
X, Ar, R, R' as under a)
R'' = H, F, Cl, Br, R', OR',

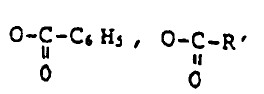

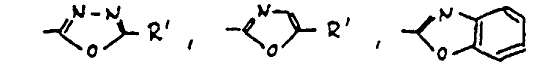

3. Photochromic substance according to claim 2 or 3, **characterised in that** it is suitable for colouring spectacle lenses made of a plastics material.

## Revendications

1. Substance photochromique à structure de diaryl-2H-naphtopyrannes qui sont liés entre eux sous la forme Y-Ar-Y, Y-Ar-Y', Y-Ar-Z, Z-Ar-Z ou Z-Ar-Z', où Y, Y', Z et Z' représentent des pyrannes.

2. Substance photochromique à structure de diaryl-2H-naphtopyrannes, caractérisée en ce qu'elle présente un substituant aryle qui réduit la migration des molécules dans des matières synthétiques, obtenue par :
a) Synthèse d'une cétone à partir d'un diarylalcane et/ou d'un éther de diaryle et d'un halogénure d'acide arylcarboxylique par acylation de Friedel-Crafts selon le schéma réactionnel suivant : 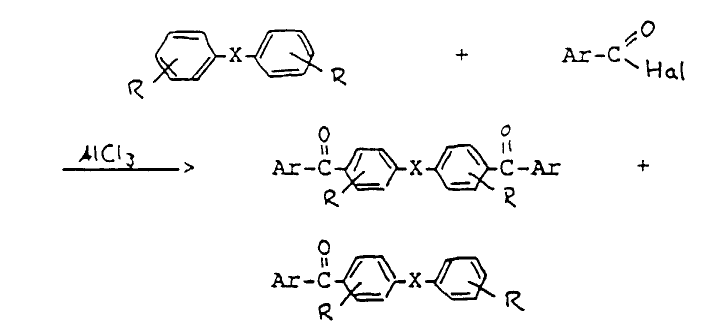 avec :
X = (CH₂)ₙ- n = 0, 1, 2, ..., 6
- (CH₂)ₙ-O- (CH₂)ₘ- n = 0, 1, 2, 3
m = 0, 1, 2, 3
R = H, F, Cl, Br, phényle, benzyle, OR', R', NR'₂
R'= alkyle ayant jusqu'à 6 atomes de carbone ou cyclo-alkyle ayant 5 ou 6 atomes de carbone
Ar = 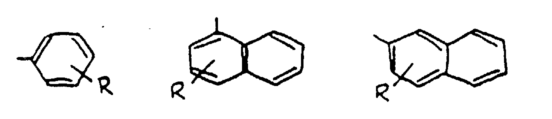
b) Synthèse d'un alcool tertiaire à triple liais carbone-carbone à partir de cétone obtenue en a) et d' acétylure de métal par addition nucléophile ;
c) Synthèse de la substance photochromique par condensation de l'alcool tertiaire avec un alcool diarylique substitué d'après le schéma réactionnel suivant : 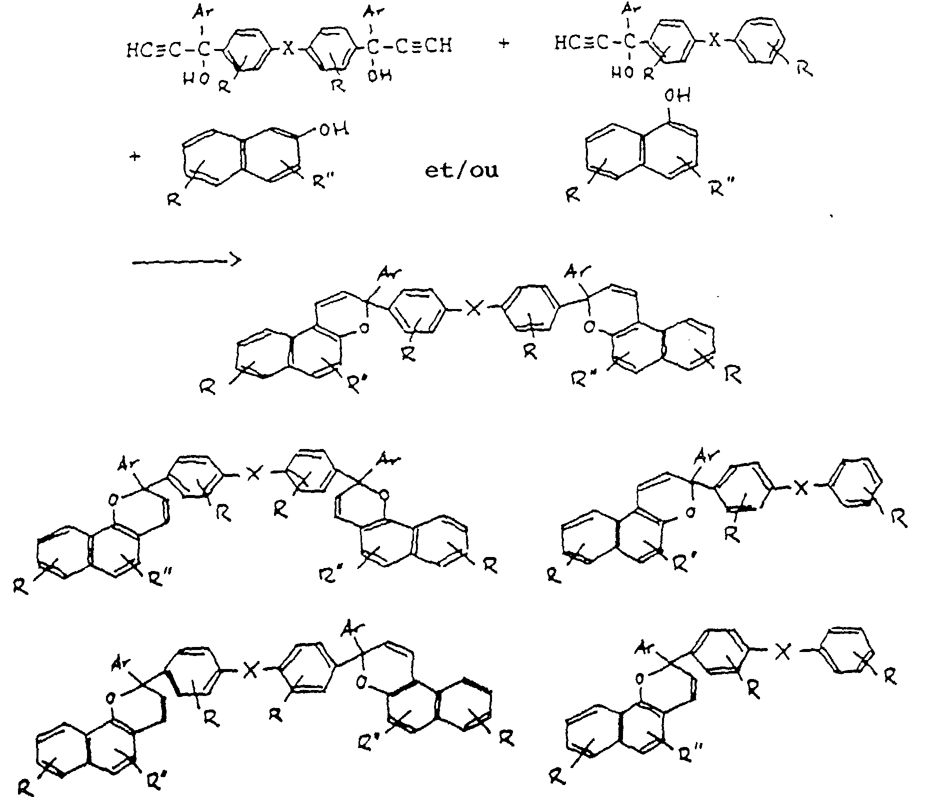 avec :
X, Ar, R, R' comme en a)
R" = H, F, Cl, Br, R', OR',

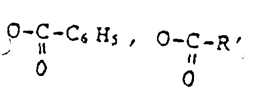

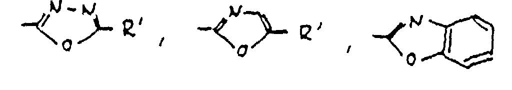

3. Substance photochromique suivant la revendication 2 ou 3, caractérisée en ce qu'elle convient pour la coloration de verres de lunettes en un matériau synthétique.
